# FASCICULE DE BREVET EUROPEEN

(11) **EP 0 497 662 B1**
(45) Date de publication et mention de la délivrance du brevet: **17.05.1995**
(21) Numéro de dépôt: 92400179.5
(22) Date de dépôt: 23.01.1992
(51) Int. Cl.: C07C 51/09, C07C 51/41, C07C 27/02

(54) **Procédé de fabrication de téréphtalate de métal alcalin ou alcalino-terreux ou de l'acide téréphtalique, de pureté élevée, à partir de polytéréphtalate de polyol et en particulier à partir de déchets d'un polytéréphtalate d'éthylène glycol**
Verfahren zur Herstellung von hochreinem Alkalimetall- oder Erdalkalimetallterephthalat oder von Terephthalsäure aus dem Polyolterephthalat, insbesondere aus dem Ethylenglykolpolyterephthalatabfall
Process for fabricating alkali metal or alkali earth metal terephtalate or of terephtalic acid having elevated purity from polyol polyterephtalate, in particular from a polyterephtalate ethylene glycol waste

(30) Priorité: 30.01.1991 FR 9101025
(43) Date de publication de la demande: 05.08.1992
(73) Titulaire: INSTITUT FRANCAIS DU PETROLE, 92502 Rueil-Malmaison (FR); Benzaria, Jacques Raphäel, 60230 Chambly (FR)
(72) Inventeur: Benzaria, Jacques, F-60230 Chambly (FR)
(74) Mandataire: Andreeff, François

(56) Documents cités:
- GB-A- 822 834
- US-A- 4 578 510

## Description

L'invention concerne un procédé de fabrication d'un sel de métal alcalin ou alcalino-terreux de l'acide téréphtalique, ou de l'acide téréphtalique lui-même, dans lequel on fait réagir un polytéréphtalate de polyol avec un hydroxyde de métal alcalin ou alcalino-terreux, caractérisé en ce que la réaction est effectuée à 130-190° C en l'absence d'eau ou en présence d'eau en quantité au plus égale, en poids, à celle de l'hydroxyde de métal alcalin ou alcalino-terreux, la proportion d'hydroxyde de métal alcalin ou alcalino-terreux représentant 50 à 300 % de la stoechiométrie par rapport au polytéréphtalate, calculée pour produire le téréphtalate entièrement salifié, la réaction étant éventuellement suivie d'une acidification si l'acide téréphtalique est le produit désiré.

D'une façon plus détaillée, l'invention concerne la fabrication de téréphtalates de métaux alcalins ou alcalino-terreux à partir de polytéréphtalate de polyol et en particulier à partir de polytéréphtalate de glycol (P.E.T.).

Les difficultés de recyclage des polyesters, type P.E.T., sont dues principalement à la coloration et aux matières étrangères se trouvant mèlées à ces produits, ainsi qu'à l'élimination des traces d'eau.

Une des meilleures formes de récupération est une dépolymérisation et récupération de l'acide téréphtalique et glycol sous forme de matières premières pouvant conduire à de nouveaux polyesters de la même pureté que les produits fabriqués à partir de matières premières neuves.

La décomposition du P.E.T. et des polyesters ayant l'acide téréphtalique comme acide dibasique est décrite dans l'art antérieur comme s'effectuant dans une solution aqueuse de soude contenant de 3 à 20 % en poids de soude, à une pression de 1,38 à 2,07 MPa (200 à 300 PSI) et à une température comprise entre 180 et 320° C, de préférence entre 210 et 250° C (US - 3,317,519).

Ce procédé conduit à des installations discontinues, à l'utilisation importante d'énergie, ainsi qu'à un matériel devant résister à la pression.

Les durées d'hydrolyse sont longues, de 3 à 5 heures, et la quantité de soude utilisée est de l'ordre de 1,5 par rapport à la théorie.

Selon une autre proposition (GB-A-822.834), on cherche à obtenir un relargage du sel d'acide téréphtalique soit en opérant à 100° C avec une solution aqueuse relativement diluée d'hydroxyde de sodium soit en opérant en présence d'un alcool. La présence d'alcool dans la présente invention n'est pas nécessaire et est même considérée comme nuisible.

Le procédé de l'invention remédie à ces défauts de la technique antérieure en proposant un procédé qui peut être réalisé plus rapidement à une température plus basse, sous une pression plus basse et notamment sous la pression normale, et dans des conditions qui permettent une mise en oeuvre en continu et l'obtention d'acide téréphtalique de qualité convenant à son emploi en polymérisation. La consommation d'énergie est donc plus faible, de même qu'il est possible d'utiliser un appareillage peu coûteux puisqu'il n'a pas besoin de résister à la pression.

Un autre avantage du procédé est d'être sélectif. Ainsi lorsque certains autres polymères ou polycondensats sont présents dans la charge de départ, ceux-ci peuvent demeurer non-affectés par le traitement et donc être facilement séparés au terme de la réaction. C'est le cas, par exemple, du polyéthylène, du polypropylène et de certaines fibres naturelles, par exemple la cellulose sous forme de fibres de coton ou de papier (étiquettes en papier par exemple).

Selon une forme de réalisation particulière, un traitement simple permet d'éliminer les colorants présents dans la charge de départ.

Le procédé de l'invention consiste donc à faire réagir un polytéréphtalate de polyol avec un hydroxyde de métal alcalin ou alcalino-terreux en l'absence d'eau ou en présence d'eau en une quantité au plus égale en poids à celle de l'hydroxyde de métal alcalin ou alcalino-terreux.

On opère avantageusement entre 140 et 180° C. La pression est avantageusement égale à la pression atmosphérique normale ou voisine de celle-ci. Il n'y a pas d'avantage décisif à opérer sous pression, la vitesse de réaction étant déjà élevée à la pression normale.

La réaction est généralement rapide, le plus souvent entre 1 et 60 minutes, par exemple 15 minutes.

L'hydroxyde de métal alcalin ou alcalino-terreux peut être utilisé en proportion stoechiométrique par rapport au polytéréphtalate, c'est-à-dire en la quantité nécessaire pour produire le sel recherché. Bien qu'on puisse produire le téréphtalate mono-acide du métal choisi, on préfère produire le téréphtalate entièrement salifié, ce qui nécessite deux atomes de métal alcalin ou un atome de métal alcalino-terreux par groupe téréphtalique présent. On peut également opérer avec un excès ou un défaut d'hydroxyde, par exemple un excès ou un défaut jusqu'à 50 % ou plus. Par exemple on pourra opérer avec 50 à 300 % de la quantité théorique d'hydroxyde, de préférence 50 à 150 %.

A titre d'exemples, on peut utiliser les hydroxydes de sodium, potassium, calcium, baryum ou magnésium, ou les oxydes des mêmes métaux, en procédant à leur hydratation.

On peut opérer sans eau, par exemple par chauffage dans une extrudeuse.

Au terme de la réaction, si la quantité d'eau n'est pas suffisante pour dissoudre le téréphtalate, lorsque celui-ci est soluble dans l'eau, on ajoute utilement l'eau nécessaire pour assurer cette dissolution. Ce sera le cas, par exemple, pour les téréphtalates de sodium et de potassium. Les téréphtalates de métaux alcalino-terreux, généralement peu solubles, se séparent par précipitation.

Si l'on désire éliminer le polyol libéré par la réaction, on peut procéder par relargage du liquide de filtration en augmentant la force ionique de la solution, par exemple par addition de chlorure de sodium. Le polyol ainsi séparé peut être purifié par distillation ou par toute autre méthode connue.

Si d'autres polymères non-réactifs sont présents dans la charge, ceux-ci sont facilement séparés de la solution aqueuse de téréphtalate de métal alcalin en raison de leur insolubilité dans cette solution. C'est le cas, par exemple, des bouteilles en plastique usagées vides, souvent à base de polyéthylène, y compris les anciennes étiquettes de ces bouteilles.

Lorsqu'on désire une pureté encore plus élevée et une décoloration complète du produit, et notamment obtenir un acide téréphtalique de qualité polymérisable, l'invention propose un procédé de décoloration de cette solution par extraction au moyen d'un alcool peu soluble dans l'eau, ayant au moins 3 atomes de carbone par molécule, et de préférence avec un alcool en C3 à C8 ou mieux en C4 à C6. Le n-butanol est préféré à une température comprise entre 40 et 80° C.

On opère de préférence à une température comprise entre 40 et 80° C, et plus généralement entre 10 et 150° C. La pression peut être, par exemple, de 0,1 à 1 MPa. La quantité d'alcool représente utilement 5 à 100 % du volume de la solution traitée.

On peut ainsi obtenir une solution aqueuse de téréphtalate de métal alcalin décolorée.

Quand le sel obtenu d'acide téréphtalique est insoluble, ce qui est le cas par exemple du téréphtalate de calcium, on peut également le purifier davantage par extraction au moyen d'un alcool, par exemple un alcool de C1 à C8, de préférence en quantité représentant 5 à 100 % du poids de ce sel.

Si l'on veut obtenir l'acide téréphtalique lui-même, on peut acidifier la solution à l'aide d'un acide sulfurique, phosphorique ou chlorhydrique ou un acide organique, à un pH de 1 à 4 et de préférence de 2,5 à 3 favorable à cette opération. On recueille alors l'acide téréphtalique insoluble dans un état de pureté élevée, après un lavage éliminant les sels produits.

De préférence le procédé selon l'invention est mis en oeuvre en continu.

### EXEMPLE 1

1178 kg de support de films radiographiques en polytéréphtalate de glycol, débarrassés de l'argent et de la gélatine, sont mélangés à froid avec 500 kg de soude à 100° C sous agitation, puis on introduit dans le mélange 100 litres d'eau. Le mélange est distribué dans un échangeur à vis où la température est 160° C, la vitesse d'introduction étant calculée de telle façon que la température de 160° C soit atteinte à mi-parcours. Le produit est recueilli dans une cuve agitée dans laquelle se trouve la quantité d'eau nécessaire pour arriver à une concentration finale de 30 % en poids de téréphtalate disodique. La solution est amenée à 70° C et est extraite par 10 % en poids de butanol. L'extraction terminée, le jus est passé sur une colonne de charbon actif qui élimine le reste des impuretés et la solution décolorée est ensuite précipitée par addition d'acide sulfurique jusqu'à pH de 2,5 - 3 et l'acide téréphtalique récupéré (rendement : 1000 kg) est correctement lavé et séché.
Indice d'acide 675
Cendre 10 ppm
Solution à 5 % dans la diméthyl formamide --- couleur : apha 10
H₂O maximum 0,5 %
Pureté : 99,9 %

### EXEMPLE 2

Même procédure que dans l'exemple 1 mais utilisation de potasse (701 kg) - température 150° C - rendement 1003 kg d'acide téréphtalique.

### EXEMPLE 3

L'appareillage est le même que dans l'exemple 1.
1178 kg de support de films radiographiques en polytéréphtalate de glycol sont mélangés avec 700 kg de chaux hydratée Ca (OH)₂ ; la température de l'échangeur à vis est de 180° C ; le produit est récupéré dans une quantité d'eau correspondant à une dilution de 100 % - Le téréphtalate de chaux est insoluble, il est filtré, lavé et séché - il est ensuite décomposé en acide téréphtalique comme indiqué à l'exemple 1.

### EXEMPLE 4

1200 kg d'emballages polyester type "bouteilles" non lavés sont broyés et traités comme dans l'exemple 1 - par contre après dilution dans l'eau à une concentration de 20 % de téréphtalate de soude, on procède à une filtration permettant d'éliminer les produits insolubles ; le reste de l'opération se termine comme dans l'exemple 1.

Le procédé s'applique également avec succès au traitement d'autres polytéréphtalates, et par exemple au traitement de polytéréphtalate de butylène glycol.

## Revendications

1. Procédé de fabrication d'un sel de métal alcalin ou alcalino-terreux de l'acide téréphtalique, ou de l'acide téréphtalique lui-même, dans lequel on fait réagir un polytéréphtalate de polyol avec un hydroxyde de métal alcalin ou alcalino-terreux, caractérisé en ce que la réaction est effectuée à 130-190° C en l'absence d'eau ou en présence d'eau en quantité au plus égale, en poids, à celle de l'hydroxyde de métal alcalin ou alcalino-terreux, la proportion d'hydroxyde de métal alcalin ou alcalino-terreux représentant 50 à 300 % de la stoechiométrie par rapport au polytéréphtalate, calculée pour produire le téréphtalate entièrement salifié, la réaction étant éventuellement suivie d'une acidification si l'acide téréphtalique est le produit désiré.

2. Procédé selon la revendication 1, dans lequel on opère avec une proportion d'hydroxyde représentant 50 à 150 % de la stoechiométrie.

3. Procédé selon la revendication 1 ou 2, dans lequel on opère à 140-180° C sous une pression sensiblement égale à la pression atmosphérique.

4. Procédé selon la revendication 1, dans lequel la proportion d'hydroxyde est sensiblement égale à la proportion stoechiométrique.

5. Procédé selon l'une des revendications 1 à 4, dans lequel l'hydroxyde est un hydroxyde de métal alcalin et dans lequel, après la réaction, on ajoute suffisamment d'eau pour dissoudre le téréphtalate de métal alcalin formé, avant acidification éventuelle.

6. Procédé selon l'une des revendications 1 à 5, dans lequel, la solution aqueuse de téréphtalate de métal alcalin est soumise à une extraction par un alcool de C3 à C8, de préférence de C4 à C8, en particulier le n-butanol.

7. Procédé selon l'une des revendications 1 à 6, dans lequel le polyol est séparé de la solution aqueuse de téréphtalate de métal alcalin par relargage à l'aide d'un composé qui augmente la force ionique de la solution.

8. Procédé selon l'une quelconque des revendications 1 à 7, dans lequel le procédé est mis en oeuvre en continu.

## Claims

1. A process for the production of an alkali or alkaline-earth metal salt of terephthalic acid, or of terephthalic acid itself, wherein a poly(polyol terephthalate) is reacted with an alkali or alkaline-earth metal hydroxide, characterised in that the reaction is carried out at 130-190°C in the absence of water or in the presence of water in a quantity by weight of at most that of the alkali or alkaline-earth metal hydroxide, the proportion of alkali or alkaline-earth metal hydroxide representing 50 % to 300 % of the stoichiometric quantity with reference to the polyterephthalate, calculated so as to produce the total salt of the terephthalate, the reaction optionally being followed by acidification if terephthalic acid is to be produced.

2. A process according to claim 1, wherein the proportion of hydroxide represents 50 % to 150 % of the stoichiometric quantity.

3. A process according to claim 1 or claim 2, wherein a temperature of 140-180°C and a pressure substantially equal to atmospheric pressure is used.

4. A process according to claim 1, wherein the proportion of hydroxide is substantially equal to the stoichiometric quantity.

5. A process according to any one of claims 1 to 4, wherein the hydroxide is an alkali metal hydroxide and wherein sufficient water is added after the reaction to dissolve the alkali metal terephthalate formed before any optional acidification.

6. A process according to any one of claims 1 to 5, wherein the aqueous alkali metal terephthalate solution is extracted with a C3 to C8, preferably C4 to C8 alcohol, in particular n-butanol.

7. A process according to any one of claims 1 to 6, wherein the polyol is separated from the aqueous alkali metal terephthalate solution by salting out using a compound which increases the ionic strength of the solution.

8. A process according to any one of claims 1 to 7, wherein the process is carried out continuously.

## Patentansprüche

1. Verfahren zur Herstellung eines Alkali- oder Erdalkalimetallsalzes von Terephthalsäure oder von Terephthalsäure selbst, worin man ein Polyolpolyterephthalat mit einem Alkali- oder Erdalkalimetallhydroxid umsetzt, dadurch gekennzeichnet, daß die Umsetzung bei 130 bis 190°C in Abwesenheit von Wasser oder in Gegenwart von Wasser in einer Gewichtsmenge von größer oder gleich derjenigen des Alkali- oder Erdalkalimetallhydroxids durchführt, wobei das Verhältnis von Alkali- oder Erdalkalimetallhydroxid, berechnet zur Herstellung des vollständig versalzten Terephthalats, 50 bis 300 % der Stöchiometrie, bezogen auf das Polyterephthalat, darstellt, wobei der Umsetzung gegebenenfalls ein Sauermachen folgt, falls Terephthalatsäure das erwünschte Produkt ist.

2. Verfahren nach Anspruch 1, worin man mit einem Hydroxidverhältnis arbeitet, das 50 bis 150 % der Stöchiometrie darstellt.

3. Verfahren nach Anspruch 1 oder 2, worin man bei 140 bis 180°C unter einem Druck, der im wesentlichen gleich dem atmosphärischen Druck ist, arbeitet.

4. Verfahren nach Anspruch 1, worin das Hydroxidverhältnis im wesentlichen gleich dem stöchiometrischen Verhältnis ist.

5. Verfahren nach einem der Ansprüche 1 bis 4, worin das Hydroxid ein Alkalimetallhydroxid ist und worin man nach der Umsetzung zum Auflösen des erzeugten Alkalimetallterephthalats vor dem eventuellen Sauermachen ausreichend Wasser zufügt.

6. Verfahren nach einem der Ansprüche 1 bis 5, worin die wäßrige Lösung des Alkalimetallterephthalats einer Extrahierung durch einen C₃ bis C₈-, bevorzugt C₄ bis C₈-Alkohol, insbesondere n-Butanol, unterworfen wird.

7. Verfahren nach einem der Ansprüche 1 bis 6, worin das Polyol von der wäßrigen Lösung des Alkalimetallterephthalats durch Aussalzen mit Hilfe einer Verbindung, welche die Ionenstärke der Lösung erhöht, abgetrennt wird.

8. Verfahren nach irgendeinem der Ansprüche 1 bis 7, worin das Verfahren kontinuierlich durchgeführt wird.
